(19) Europäisches Patentamt
European Patent Office
Office européen des brevets

(11) EP 1 604 973 A1

(12) **EUROPÄISCHE PATENTANMELDUNG**

(43) Veröffentlichungstag:
**14.12.2005 Patentblatt 2005/50**

(21) Anmeldenummer: **05011538.5**

(22) Anmeldetag: **27.05.2005**

(51) Int Cl.[7]: **C07C 211/42**, C07C 211/63, C07F 15/00, B01J 31/22, C07B 53/00 // C07M7:00

(84) Benannte Vertragsstaaten:
**AT BE BG CH CY CZ DE DK EE ES FI FR GB GR HU IE IS IT LI LT LU MC NL PL PT RO SE SI SK TR**
Benannte Erstreckungsstaaten:
**AL BA HR LV MK YU**

(30) Priorität: **08.06.2004 DE 102004027772**

(71) Anmelder: **LANXESS Deutschland GmbH**
**51369 Leverkusen (DE)**

(72) Erfinder:
- **Grützmacher, Hansjörg, Professor Dr.**
**8907 Wettswil (CH)**
- **Maire, Pascal, Dr.**
**4410 Liestal (CH)**

(54) **Bistropylidendiamine und deren Verwendung**

(57) Die vorliegende Erfindung betrifft Bistropylidendiamine, ein Verfahren zu ihrer Herstellung sowie deren Verwendung in der Katalyse.

EP 1 604 973 A1

## Beschreibung

Die vorliegende Erfindung betrifft Bistropylidendiamine, ein Verfahren zu ihrer Herstellung sowie deren Verwendung in der Katalyse.

Aus Deblon (Dissertation Nr. 13920, ETH Zürich, 2000, Kapitel 5) und Maire (Dissertation Nr. 14396, ETH Zürich, 2001) ist bekannt, dass Übergangsmetallkomplexe von Olefin-Phosphanverbindungen für homogenkatalytische Reaktionen wie insbesondere Hydrierungen und Hydrosilylierungen besonders geeignet sind.

Für industrielle Anwendungen wäre es jedoch vorteilhaft, auf die oft teuren und oxidationsempfindlichen Phosphane verzichten zu können. Es bestand daher das Bedürfnis, ein Katalysatorsystem und dafür geeignete Liganden bereitzustellen, das ohne den Einsatz von Phosphanen auskommt und gute Performance in katalytischen Reaktionen zeigt.

Es wurden nun Verbindungen der Formel (I) gefunden,

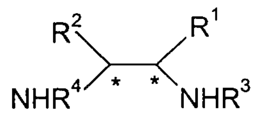

(I)

in der

| | |
|---|---|
| * | ein stereogenes Kohlenstoffatom markiert, das (S)- oder (R)-konfiguriert sein kann |
| $R^1$ und $R^2$ | jeweils unabhängig voneinander ausgewählt sind aus der Gruppe $C_1$-$C_{12}$-Alkyl, $C_1$-$C_{12}$-Alkoxy, $C_1$-$C_{12}$-Halogenalkoxy, $C_1$-$C_{12}$-Halogenalkyl, $C_4$-$C_{14}$-Aryl, $C_5$-$C_{15}$ -Arylalkyl oder zusammen stehen für $C_3$-$C_{12}$-Alkylen oder $C_3$-$C_{12}$-Alkenylen, |
| $R^3$ und $R^4$ | jeweils unabhängig voneinander für Reste der Formel (II) stehen |

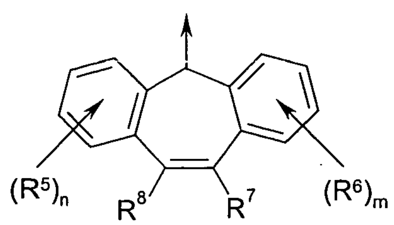

(II)

wobei

| | |
|---|---|
| die Pfeile | jeweils die Bindung des gesamten Restes zum Stickstoffatom anzeigen oder, sofern sie in die Mitte eines aromatischen Systems zeigen, eine Bindung der jeweiligen Reste zum aromatischen Gerüst in beliebiger Position, |
| $R^5$ und $R^6$ | jeweils unabhängig voneinander ausgewählt sind aus der Gruppe Fluor, Chlor, Brom, Iod, Nitro, freies oder geschütztes Formyl, $C_1$-$C_{12}$-Alkyl, $C_1$-$C_{12}$-Alkoxy, $C_1$-$C_{12}$-Halogenalkoxy, $C_1$-$C_{12}$-Halogenalkyl, $C_4$-$C_{14}$-Aryl, $C_5$-$C_{15}$-Arylalkyl oder Resten der Formel (IV), |

$$L\text{-}Q\text{-}T\text{-}W \qquad \text{(IV)}$$

in der unabhängig voneinander

| | |
|---|---|
| L | fehlt oder für $C_1$-$C_8$-Alkylen oder $C_2$-$C_8$-Alkenylen steht und |
| Q | fehlt oder für Sauerstoff, Schwefel oder $NR^9$ steht, |

T für eine Carbonyl-Gruppe steht und

W für $R^9$, $OR^9$, $NHR^9$ oder $N(R^{10})_2$ steht,

wobei $N(R^{10})_2$ weiterhin als Ganzes für einen 5 oder 6 gliedrigen cyclischen Aminorest stehen kann oder Resten der Formeln (Va-g)

| | | | |
|---|---|---|---|
| L-W | (Va) | $L-SO_2-W$ | (Vb) |
| $L-NR^{12}SO_2R^{12}$ | (Vc) | $L-SO_3Z$ | (Vd) |
| $L-PO_3Z_2$ | (Ve) | L-COZ | (Vf) |
| L-CN | (Vg) | | |

in denen L, Q, W und $R^{10}$ die unter der Formel (IV) angegebene Bedeutung besitzen und Z für Wasserstoff oder M steht und

$R^7$ und $R^8$ jeweils unabhängig voneinander stehen für Wasserstoff, Cyano, Fluor, Chlor, Brom, Iod, $C_1$-$C_{18}$-Alkyl, $C_4$-$C_{24}$-Aryl, $C_5$-$C_{25}$-Arylalkyl, $CO_2M$, wobei M für ein Alkalimetallion oder ein gegebenenfalls organisches Ammoniumion stehen kann, $CONH_2$, $SO_2N(R^9)_2$, wobei $R^9$ für Wasserstoff, $C_1$-$C_{12}$-Alkyl, $C_4$-$C_{14}$-Aryl oder $C_5$-$C_{15}$-Arylalkyl steht, $SO_3M$ oder für Reste der Formel (III),

$$T\text{-}Het\text{-}R^{10}, \qquad \text{(III)}$$

in der

T T fehlt oder für Carbonyl steht,

H Het für Sauerstoff oder $NR^9$ steht,

$R^{10}$ für $C_1$-$C_{18}$-Alkyl, $C_4$-$C_{24}$-Aryl oder $C_5$-$C_{25}$-Arylalkyl oder $N(R^{10})_2$ als Ganzes für einen 5 oder 6 gliedrigen cyclischen Aminorest steht und

n und m jeweils unabhängig voneinander für 0, 1, 2 oder 3 stehen.

Die Verbindungen der Formel (I) sind chiral. Die Erfindung umfasst alle auftretenden Stereoisomeren als solche, sowie beliebige Mischungen davon. Die Begriffe stereoisomerenangereichert (enantiomerenangereichert bzw. diastereomerenangereichert) bedeuten im Rahmen der Erfindung stereoisomerenreine (enantiomerenreine bzw. diastereomerenreine) Verbindungen oder Mischungen von Stereoisomeren (Enantiomeren bzw. Diastereomeren), in denen ein Stereoisomeres (Enantiomeres bzw. Diastereomeres) in einem größeren Anteil vorliegt als ein anderes bzw. das andere. Stereoisomerenangereichert bedeutet beispielsweise und bevorzugt einen Gehalt eines Stereoisomeren von 50 % bis 100 Gew.-%, besonders bevorzugt 70 % bis 100 Gew.-% und ganz besonders bevorzugt 90 bis 100 Gew.-%, bezogen auf die Summe der jeweiligen Stereoisomeren.

Der Rahmen der Erfindung umfasst alle Kombinationen der oben stehenden und im Folgenden aufgeführten, allgemeinen oder in Vorzugsbereichen genannten Restedefinitionen, Parameter und Erläuterungen untereinander, also auch beliebige Kombinationen zwischen den jeweiligen Bereichen und Vorzugsbereichen.

**Aryl** steht im Rahmen der Erfindung, sofern nicht gesondert angegeben, für carbocyclische aromatische Reste wie vorzugsweise Phenyl, Naphtyl, Phenanthrenyl und Anthracenyl, oder für heteroaromatische Reste, in denen keines, ein, zwei oder drei Gerüstkohlenstoffatome pro Cyclus, im gesamten Molekül mindestens jedoch ein Gerüstkohlenstoffatom durch Heteroatome substituiert sind, die ausgewählt sind aus der Gruppe Stickstoff, Schwefel oder Sauerstoff, wie vorzugsweise Pyridinyl, Oxazolyl, Thiophen-yl, Benzofuranyl, Benzothiophen-yl, Dibenzofuranyl, Dibenzothiophen-yl, Furanyl, Indolyl, Pyridazinyl, Pyrazinyl, Imidazolyl, Pyrimidinyl und Chinolinyl.

Weiterhin können die carbocyclischen, aromatischen Reste oder heteroaromatischen Reste mit bis zu fünf gleichen oder verschiedenen Substituenten pro Cyclus substituiert sein.

Beispielsweise und bevorzugt sind die Substituenten ausgewählt aus der Gruppe Brom, Fluor, Chlor, Nitro, Cyano, freies oder geschütztes Formyl, freies oder geschütztes Hydroxy, $C_1$-$C_{12}$-Alkyl, $C_1$-$C_{12}$-Halogenalkyl, $C_1$-$C_{12}$-Alkoxy, $C_1$-$C_{12}$-Halogenalkoxy, $C_4$-$C_{14}$-Aryl wie zum Beispiel Phenyl, $C_5$-$C_{15}$-Arylalkyl wie zum Beispiel Benzyl, Di ($C_1$-$C_{12}$-alkyl)-amino, ($C_1$-$C_{12}$-Alkyl)-amino, CO($C_1$-$C_{12}$-Alkyl), OCO($C_1$-$C_{12}$-Alkyl), NHCO($C_1$-$C_{12}$-Alkyl), N($C_1$-$C_8$-Al-

kyl)CO($C_1$-$C_{12}$-Alkyl), CO($C_4$-$C_{14}$-Aryl), OCO($C_4$-$C_{14}$-Aryl), NHCO($C_4$-$C_{14}$-Aryl), N($C_1$-$C_8$-Alkyl)CO($C_4$-$C_{14}$-Aryl), COO-($C_1$-$C_{12}$-Alkyl), COO-($C_4$-$C_{14}$-Aryl), CON($C_1$-$C_{12}$-Alkyl)$_2$ oder CONH($C_1$-$C_{12}$-Alkyl) $CO_2M$, $CONH_2$, $SO_2NH_2$, $SO_2N$($C_1$-$C_{12}$-Alkyl)$_2$, $SO_3M$ wobei M jeweils für gegebenenfalls substituiertes Ammonium, Lithium, Natrium oder Kalium steht.

Beispielsweise und bevorzugt steht Aryl für Phenyl oder Naphthyl, das mit keinem, einem, zwei oder drei Resten pro Cyclus weiter substituiert sein kann, die ausgewählt sind aus der Gruppe Fluor, Chlor, Cyano, $C_1$-$C_8$-Alkyl, $C_1$-$C_8$-Perfluoralkyl, $C_1$-$C_8$-Alkoxy, Phenyl, Benzyl, Di($C_1$-$C_{12}$-alkyl)-amino, CO($C_1$-$C_{12}$-Alkyl), COO-($C_1$-$C_{12}$-Alkyl), CON($C_1$-$C_{12}$-Alkyl)$_2$ oder $SO_2N$($C_1$-$C_{12}$-Alkyl)$_2$.

Besonders bevorzugt steht Aryl für Phenyl, das mit keinem, einem oder zwei Resten pro Cyclus weiter substituiert sein kann, die ausgewählt sind aus der Gruppe Fluor, Chlor, Cyano, $C_1$-$C_4$-Alkyl, $C_1$-$C_4$-Perfluoralkyl, $C_1$-$C_4$-Alkoxy, Phenyl oder $SO_2N$($C_1$-$C_4$-Alkyl)$_2$.

Analog gelten die Definition und die Vorzugsbereiche im Rahmen der Erfindung auch für Aryloxy-Substituenten und den Arylteil eines Arylalkyl-Restes.

**Geschütztes Formyl** steht im Rahmen der Erfindung, sofern nicht gesondert angegeben, für einen Formyl-Rest, der durch Überführung in ein Aminal, Acetal oder ein gemischtes Aminalacetal geschützt ist, wobei die Aminale, Acetale und gemischten Aminalacetale acyclisch oder cyclisch sein können.

Beispielsweise und bevorzugt steht geschütztes Formyl für einen 1,1-(2,4-Dioxycyclopentandiyl)-Rest.

**Geschütztes Hydroxy** steht im Rahmen der Erfindung, sofern nicht gesondert angegeben, für einen Hydroxy-Rest, der durch Überführung in ein Ketal, Acetal oder ein gemischtes Aminalacetal geschützt ist, wobei die Acetale und gemischten Aminalacetale acyclisch oder cyclisch sein können.

Beispielsweise und bevorzugt steht geschütztes Hydroxy für einen Tetrahydropyranyl-Rest (O-THP).

**Alkyl** bzw. **Alkylen,** bzw. **Alkoxy** bzw. **Alkenyl** bzw. **Alkenylen** steht im Rahmen der Erfindung, sofern nicht gesondert angegeben, für einen geradkettigen, cyclischen, verzweigten oder unverzweigten Alkyl- bzw. Alkylen- bzw. Alkoxy- bzw. Alkenyl- bzw. Alkenylen-Rest, der gegebenenfalls durch $C_1$-$C_4$-Alkoxy in der Art weiter substituiert sein kann, dass jedes Kohlenstoffatom des Alkyl- bzw. Alkylen-, bzw. Alkoxy bzw. Alkenyl- bzw. Alkenylen-Restes höchstens ein Heteroatom ausgewählt aus der Gruppe Sauerstoff, Stickstoff oder Schwefel trägt.

Gleiches gilt analog für den Alkylenteil eines **Arylalkyl**-Restes.

Beispielsweise steht im Rahmen der Erfindung $C_1$-$C_4$-Alkyl bevorzugt für Methyl, Ethyl, 2-Ethoxyethyl, n-Propyl, Isopropyl, n-Butyl, tert.-Butyl, $C_1$-$C_8$-Alkyl darüber hinaus beispielsweise für n-Pentyl, Cyclohexyl, n-Hexyl, n-Heptyl, n-Octyl oder iso-Octyl, $C_1$-$C_{12}$-Alkyl, weiter darüber hinaus z.B. für Norbornyl, Adamantyl, n-Decyl und n-Dodecyl und $C_1$-$C_{18}$-Alkyl noch weiter darüber hinaus für n-Hexadecyl und n-Octadecyl.

Beispielsweise steht im Rahmen der Erfindung $C_1$-$C_8$-Alkylen bevorzugt für Methylen, 1,1-Ethylen, 1,2-Ethylen, 1,1-Propylen, 1,2-Propylen, 1,3-Propylen, 1,1-Butylen, 1,2-Butylen, 2,3-Butylen und 1,4-Butylen, 1,5-Pentylen, 1,6-Hexylen, 1,1-Cyclohexylen, 1,4-Cyclohexylen, 1,2-Cyclohexylen und 1,8-Octylen.

Beispielsweise steht im Rahmen der Erfindung $C_1$-$C_4$-Alkoxy bevorzugt für Methoxy, Ethoxy, Isopropoxy, n-Propoxy, n-Butoxy und tert.-Butoxy, $C_1$-$C_8$-Alkoxy darüber hinaus für Cyclohexyloxy.

Beispielsweise steht im Rahmen der Erfindung $C_2$-$C_8$-Alkenyl bevorzugt für Allyl, 3-Propenyl und 4-Butenyl.

Beispielsweise steht im Rahmen der Erfindung $C_3$-$C_8$-Alkenylen bevorzugt für 2-Butendiyl.

**Halogenalkyl** bzw. **Halogenalkoxy,** steht im Rahmen der Erfindung, sofern nicht gesondert angegeben; für einen geradkettigen, cyclischen, verzweigten oder unverzweigten Alkyl- bzw. Alkoxy-Rest, der durch Halogenatome einfach, mehrfach oder vollständig substituiert ist. Reste die vollständig durch Fluor substituiert sind, werden mit **Perfluoralkyl** bzw. **Perfluoralkoxy** bezeichnet.

Beispielsweise steht im Rahmen der Erfindung $C_1$-$C_{12}$-Halogenalkyl für Trifluormethyl, 2,2,2-Trifluorethyl, Chlormethyl, Fluormethyl, Brommethyl, 2-Bromethyl, 2-Chlorethyl, Nonafluorbutyl, n-Perfluoroctyl oder n-Perfluordodecyl.

Im Folgenden werden die bevorzugten Substitutionsmuster für Verbindungen der Formel (I) definiert:

- * bezeichnet bevorzugt jeweils identisch konfigurierte stereogene Kohlenstoffatome d.h. eine (S),(S)- oder (R),(R)-Konfiguration.
- $R^1$ und $R^2$ sind bevorzugt jeweils unabhängig voneinander ausgewählt aus der Gruppe $C_1$-$C_{12}$-Alkyl, $C_4$-$C_{14}$-Aryl oder stehen zusammen für $C_3$-$C_{12}$-Alkylen und sind besonders bevorzugt identisch ausgewählt aus der Gruppe $C_1$-$C_4$-Alkyl, Phenyl oder stehen zusammen für 1,4-Butylen.
- $R^3$ und $R^4$ stehen bevorzugt jeweils unabhängig voneinander für Reste der Formel (II), in der

  $R^5$ und $R^6$ jeweils unabhängig voneinander für Wasserstoff, Fluor, Iod, $C_1$-$C_{12}$ -Alkyl, $C_1$-$C_{12}$-Alkoxy, $C_4$-$C_{10}$-Aryl oder für Reste der Formel (IV) stehen, in der wiederum T für Carbonyl und Het für Sauerstoff oder $NR^9$ steht und

  in der

  n und m jeweils unabhängig voneinander für 0 oder 1 stehen und in der

$R^7$ und $R^8$ jeweils unabhängig voneinander ausgewählt sind aus der Gruppe Fluor, Brom, Nitro, $C_1$-$C_4$-Alkyl, $C_1$-$C_4$-Alkoxy oder Resten der Formeln (Vb) und (Vg),

$R^3$ und $R^4$ stehen besonders bevorzugt für einen Rest der Formel (II), in der

$R^5$ und $R^6$ jeweils unabhängig voneinander für Wasserstoff, $C_1$-$C_{12}$-Alkyl, $C_1$-$C_{12}$-Alkoxy, $C_4$-$C_{10}$-Aryl

n und m jeweils identisch für 0 oder 1 stehen und

$R^7$ und $R^8$ jeweils identisch ausgewählt sind aus der Gruppe Fluor oder Resten der Formeln (Vb) und (Vg),

$R^3$ und $R^4$ stehen ganz besonders bevorzugt für einen Rest der Formel (II), in der

$R^5$ und $R^6$ jeweils unabhängig voneinander für Wasserstoff, $C_1$-$C_{12}$-Alkyl, $C_1$-$C_{12}$-Alkoxy oder $C_4$-$C_{10}$-Aryl und

n und m jeweils für 0 stehen.

Von Verbindungen der Formel (I) sind solche ganz besonders bevorzugt, die an den Stickstoffatomen Reste tragen, die ausgewählt sind aus der Gruppe

10-Cyano-5*H*-dibenzo[a,d]cyclohepten-5-yl ($^{CN}$trop), 5*H*-dibenzo[a,d]cyclohepten-5-yl (trop), 10-Methyl-5*H*-dibenzo[a,d]cylohepten-5-yl ($^{Me}$trop),10-Methoxy-5*H* dibenzo[a,d]cyclohepten-5-yl ($^{MeO}$trop), 10-Phenyl-5*H*-dibenzo[a,d]cyclohepten-5-yl ($^{Ph}$trop), 10,11-Diphenyl-5*H*-dibenzo[a,d]cyclohepten-5-yl ($^{Ph2}$trop) [(5S)-10-[(-)-Menthyloxy]-5*H*-dibenzo[a,d]cyclohepten-5-yl], (S-$^{Menthyloxy}$trop) und [(5R)-10-[(-)-Menthyloxy]-5*H*-dibenzo[a,d]cyclohepten-5-yl] (R-$^{Menthyloxy}$trop).

Zur Herstellung von Verbindungen der Formel (I) geht man vorzugsweise so vor, dass Verbindungen der Formel (VI)

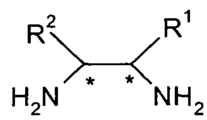

(VI)

in der $R^1$ und $R^2$ die vorstehend genannte Bedeutung besitzen

gegebenenfalls in Gegenwart von organischem Lösungsmittel mit Verbindungen der Formel (VII) umgesetzt werden,

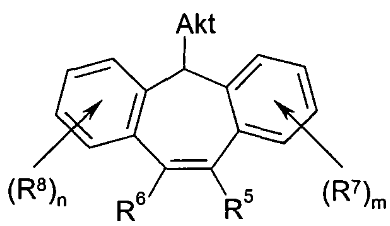

(VII)

in der $R^5$, $R^6$, $R^7$, $R^8$, n und m die vorstehend genannten Bedeutungen besitzen und Akt für Chlor, Brom, Iod, Trifluoracetyl oder einen Sulfonyloxy-Rest steht, die dabei entstehenden Ammoniumsalze der Formeln (VIIIa) und/oder (VIIIIb) und/oder (VIIIc)

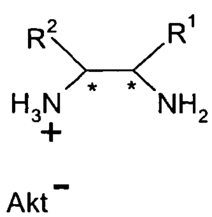

(VIIIa)

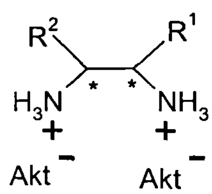

(VIIIb)

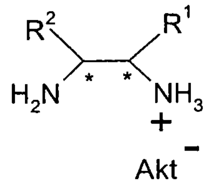

(VIIIc)

in Gegenwart von Base entweder in situ oder in einem nachgeschalteten Reaktionsschritt in Verbindungen der Formel (I) überführt werden.

Die Verbindungen der Formel (VII) sind entweder literaturbekannt oder analog zur Literatur synthetisierbar.

Die Umsetzung kann gegebenenfalls und wird bevorzugt in Gegenwart von organischem Lösungsmittel durchgeführt. Als organische Lösungsmittel sind beispielsweise geeignet:

Aliphatische oder aromatische, gegebenenfalls halogenierte Kohlenwasserstoffe, wie beispielsweise verschiedene Benzine, Benzol, Toluol, Xylol, Chlorbenzol, Dichlorbenzol, verschiedene Petrolether, Hexan, Cyclohexan, Dichlormethan, Chloroform, Tetrachlorkohlenstoff; Ether, wie Diethylether, Methyl-tert.-butylether, Diisopropylether, Dioxan, Tetrahydrofuran oder Ethylenglykoldimethyl- oder -diethylether oder Gemische solcher organischen Lösungsmittel. Bevorzugt ist Dichlormethan.

Als Basen sind beispielsweise geeignet: Erdalkali- oder Alkalimetallhydride, -hydroxide, -amide, alkylsubstituierte -disilylamide, -dialkylamide, -alkoholate, oder -carbonate wie beispielsweise Natriumhydrid, Natriumamid, Lithium-diethylamid, Natriummethylat, Natriumbistrimethylsilylamid, Natriumethylat, Kalium-tert.-butylat, Natriumhydroxid, Kaliumhydroxid, Natriumcarbonat und Kaliumcarbonat, tertiäre Amine, wie Trimethylamin, Triethylamin, Tributylamin, Trioctylamin, Di-isopropyl-ethylamin, Tetramethylguanidin, N,N-Dimethylanilin, Diazabicyclooctan (DABCO), Diazabicyclononen (DBN) oder Diazabicycloundecen (DBU), Piperidin und N-Methylpiperidin. Bevorzugt ist Kaliumcarbonat.

Sowohl das Herstellungsverfahren für die Verbindungen der Formel (I) als auch die Verbindungen der Formel (VIII) als unverzichtbare Intermediate sind von der Erfindung vollumfänglich umfasst.

Die Erfindung schließt auch Übergangsmetallkomplexe von Verbindungen der Formel (I) ein sowie Katalysatoren ein, die die erfindungsgemäßen Übergangsmetallkomplexe von Verbindungen der Formel (I) enthalten.

Bevorzugte Übergangsmetallkomplexe sind Übergangsmetallkomplexe von Ruthenium, Osmium, Cobalt, Rhodium, Iridium, Nickel, Palladium, Platin und Kupfer, bevorzugt solche von Ruthenium, Rhodium, Iridium, Nickel, Palladium und Platin, besonders bevorzugt solche von Rhodium und Iridium.

Als Katalysatoren können beispielsweise isolierte Übergangsmetallkomplexe eingesetzt werden, die beispielsweise aus den Verbindungen der Formel (I) und einer Metallverbindung erzeugt wurden, oder Übergangsmetallkomplexe, die aus den Verbindungen der Formel (I) und einer Metallverbindung im Reaktionsmedium der Katalyse erzeugt werden.

Geeignete Metallverbindungen sind beispielsweise und bevorzugt solche der Formel (IXa)

$$M^1(Y^1)_p \qquad \text{(IXa)}$$

in der

$M^1$ für Ruthenium, Rhodium, Iridium, Nickel, Palladium, Platin oder Kupfer und

$Y^1$ für Chlorid, Bromid, Acetat, Nitrat, Methansulfonat, Trifluormethansulfonat oder Acetylacetonat und

p im Falle von Ruthenium, Rhodium und Iridium für 3, im Falle von Nickel, Palladium und Platin für 2 und im Falle von Kupfer für 1 steht,

oder Metallverbindungen der allgemeinen Formel (IXb)

$$M^1(Y^2)_pB^1_2 \qquad \text{(IXb)}$$

in der

$Y^2$ für ein Anion wie z.B. Chlorid, Bromid, Acetat, Methansulfonat, Trifluormethansulfonat, Tetrafluoroborat, Hexafluorophosphat, Perchlorat, Hexafluoroantimonat, Tetra(bis-3,5 trifluormethylphenyl)borat oder Tetraphenylborat steht und

p im Falle von Rhodium und Iridium für 1, im Falle von Nickel, Palladium, Platin und Ruthenium für 2 und im Falle von Kupfer für 1 steht,

$B^1$ jeweils für ein $C_2$-$C_{12}$-Alken wie beispielsweise Ethylen oder Cycloocten, oder ein Nitril wie beispielsweise Acetonitril, Benzonitril oder Benzylnitril steht, oder

$B^1{}_2$ zusammen für ein ($C_4$-$C_{12}$)-Dien wie beispielsweise Norbornadien oder 1,5-Cyclooctadien steht

oder Metallverbindungen der Formel (IXc)

$$[M^2B^2Y^1{}_2]_2 \qquad (IXc)$$

in der

$M^2$ für Ruthenium und

$B^2$ für Arylreste wie zum Beispiel Cymol, Mesityl, Phenyl oder Cyclooctadien, Norbornadien oder Methylallyl steht

oder Metallverbindungen der Formel (IXd)

$$Me_p[M^3(Y^3)_4] \qquad (IXd),$$

wobei

$M^3$ für Palladium, Nickel, Iridium oder Rhodium und

$Y^3$ für Chlorid oder Bromid steht und

Me für Lithium, Natrium, Kalium, Ammonium oder organisches Ammonium steht und

p im Falle von Rhodium und Iridium 3, im Falle von Nickel, Palladium und Platin für 2 steht,

oder Metallverbindungen der Formel (IXe)

$$[M^4(B^3)_2]An \qquad (IXe),$$

wobei

$M^4$ für Iridium oder Rhodium und

$B^3$ für ein ($C_4$-$C_{12}$)-Dien wie beispielsweise Norbornadien oder 1,5-Cyclooctadien steht

An für ein nicht oder schwach koordinierendes Anion wie zum Beispiel Methansulfonat, Trifluormethansulfonat, Tetrafluoroborat, Hexafluorophosphat, Perchlorat, Hexafluoroantimonat, Tetra(bis-3,5-trifluormethylphenyl)borat oder Tetraphenylborat steht.

Darüberhinaus sind als Metallverbindungen beispielsweise Ni(1,5-Cyclooctadien)$_2$, Pd$_2$(dibenzylidenaceton)$_3$, Pd[PPh$_3$]$_4$ Cylopentadienyl$_2$Ru, Rh(acac)(CO)$_2$, [RhCl(CO)$_2$]; Ir(pyridin)$_2$(1,5-Cyclooctadien), Ir(acac)(CO)$_2$,

$[IrCl(CO)_2]$, Cu(Phenyl)Br, Cu(Phenyl)Cl, Cu(Phenyl)I, $Cu(PPh_3)_2Br$, $[Cu(CH_3CN)_4]BF_4$ und $[Cu(CH_3CN)_4]PF_6$ oder mehrkernige verbrückte Komplexe wie beispielsweise $[Rh(COD)Cl]_2$ und $[Rh(COD)Br]_2$, $[Rh(Ethen)_2Cl]_2$, $[Rh(Cylooc-ten)_2Cl]_2$ geeignet.

Bevorzugt werden als Metallverbindungen eingesetzt:
$[Rh(COD)Cl]_2$, $[Rh(COD)_2Br]$, $[Rh(COD)_2]ClO_4$, $[Rh(COD)_2]BF_4$, $[Rh(COD)_2]PF_6$, $[Rh(COD)_2]OTf$, $[Rh(COD)_2]BAr_4$ (Ar = 3,5-bistrifluormethylphenyl) $[Rh(COD)_2]SbF_6$ $RuCl_2(COD)$, $[(Cymol)RuCl_2]_2$, $[(Benzol)RuCl_2]_2$, $[(Mesitylen)RuCl_2]_2$, $[(Cymol)RuBr_2]_2$, $[(Cymol)RuI_2]_2$, $[(Cymol)Ru(BF_4)_2]_2$, $[(Cymol)Ru(PF_6)_2]_2$, $[(Cymol)Ru(BAr_4)_2]_2$, (Ar = 3,5-bistrifluormethylphenyl), $[(Cymol)Ru(SbF_6)_2]_2$, $[Ir(cod)_2Cl]_2$, $[Ir(COD)_2]PF_6$, $[Ir(COD)_2]ClO_4$, $[Ir(COD)_2]SbF_6$ $[Ir(COD)_2]BF_4$, $[Ir(COD)_2]OTf$, $[Ir(COD)_2]BAr_4$ (Ar = 3,5-bistrifluormethylphenyl), $RuCl_3$, $NiCl_2$, $RhCl_3$, $PdCl_2$, $PdBr_2$, $Pd(OAc)_2$, $Pd_2(dibenzylidenaceton)_3$, $Pd(acetylacetonat)_2$, $Rh(acetylacetonat)(CO)_2$, $[RhCl(CO)_2]$; $Ir(pyridin)_2$(1,5-Cyclooctadien), $Ir(acetylacetonat)(CO)_2$, $[IrCl(CO)_2]$ $[Rh(nbd)Cl]_2$, $[Rh(nbd)_2Br]$, $[Rh(nbd)_2]ClO_4$, $[Rh(nbd)_2]BF_4$, $[Rh(nbd)_2]PF_6$, $[Rh(nbd)_2]OTf$, $[Rh(nbd)_2]BAr_4$ (Ar = 3,5-bistrifluormethylphenyl) $[Rh(nbd)_2]SbF_6$ $RuCl_2(nbd)$, $[Ir(nbd)_2]PF_6$, $[Rh(nbd)_2]ClO_4$, $[Rh(nbd)_2]BF_4$, $[Rh(nbd)_2]PF_6$, $[Rh(nbd)_2]OTf$, (Ar = 3,5-bistrifluormethylphenyl), $Ir(pyridin)_2(nbd)$, $[Ru(DMSO)_4Cl_2]$, $[Ru(CH_3CN)_4Cl_2]$, $[Ru(PhCN)_4Cl_2]$, $[Ru(COD)Cl_2]_n$, $[Ru(COD)(Methallyl)_2]$, $[Ru(acetylacetonat)_3]$

Noch weiter bevorzugt sind $Rh(acetylacetonat)(CO)_2$, $[RhCl(CO)_2]$ und $Ir(acetylacetonat)(CO)_2$, $[IrCl(CO)_2]$, $[Rh(nbd)_2]ClO_4$, $[Rh(nbd)_2]BF_4$, $[Rh(nbd)_2]PF_6$, $[Rh(nbd)_2]OTf$, $[Ir(nbd)_2]ClO_4$, $[Ir(nbd)_2]BF_4$, $[Ir(nbd)_2]PF_6$, $[Ir(nbd)_2]OTf$, $[Ir(nbd)_2]ClO_4$, $[Ir(nbd)_2]BF_4$, $[Ir(nbd)_2]PF_6$, $[Ir(nbd)_2]OTf$, $[Ir(nbd)_2]ClO_4$, $[Ir(nbd)_2]BF_4$, $[Ir(nbd)_2]PF_6$ und $[Ir(nbd)_2]OTf$,

Die Menge der eingesetzten Metallverbindung kann bezogen auf den Metallgehalt beispielsweise 25 bis 200 mol-% im Bezug auf die eingesetzte Verbindung der Formel (I) betragen, bevorzugt sind 80 bis 140 mol-%, ganz besonders bevorzugt 90 bis 120 mol-% und noch weiter bevorzugt 95 bis 105 mol-% .

Ganz besonders bevorzugte Übergangsmetallkomplexe von Verbindungen der Formel (I) sind solche die der Formel (Xa), (Xb) oder(Xc) gehorchen

$$[M^5(I)]An \qquad (Xa)$$

$$[M^5(I)(CO)Hal]An \qquad (Xb)$$

$$[Ir(I)(Diolefin)]An \qquad (Xc)$$

in denen jeweils

$M^5$ für Rhodium oder Iridium

(I) für eine Verbindung der Formel (I)

Diolefin für ein Dien wie beispielsweise 1,5-Cyclooctadien oder Norbornadien und

An ohne Rücksicht auf mögliche Koordination an das Metallatom für ein Anion wie beispielsweise Chlorid, Bromid, Iodid, Methansulfonat, Trifluormethansulfonat, Tetrafluoroborat, Hexafluorophosphat, Perchlorat, Hexafluoroantimonat, Tetra(bis-3,5-trifluormethylphenyl)borat oder Tetraphenylborat und

Hal für Chlorid, Bromid oder Iodid steht.

Die Katalysatoren, die in situ erzeugten Übergangsmetallkomplexe oder isolierten Übergangsmetallkomplexe enthalten, eignen sich insbesondere für den Einsatz in der homogenen Katalyse.

Bevorzugt werden die erfindungsgemäßen Katalysatoren für Hydrogenierungen eingesetzt, besonders bevorzugt für asymmetrische Hydrogenierungen, sofern die Verbindungen unter eingangs beschriebenen Voraussetzungen chiral sind.

Bevorzugte Hydrogenierungen sind beispielsweise Hydrogenierungen von prochiralen C=C-Bindungen wie zum Beispiel prochiralen Enaminen, Olefinen, Enolethern, C=O-Bindungen wie zum Beispiel prochiralen Ketonen und C=N-Bindungen wie zum Beispiel prochiralen Iminen. Besonders bevorzugte asymmetrische Hydrogenierungen sind Hydrogenierungen von C=O-Bindungen wie zum Beispiel prochiralen Ketonen.

In einer bevorzugten Ausführungsform wird die Hydrogenierung in Gegenwart eines Wasserstoffspendermoleküls und gegebenenfalls einer Base durchgeführt.

Wasserstoffspendermoleküle sind beispielsweise molekularer Wasserstoff, Ameisensäure, Ethanol oder Isopropanol, Basen beispielseweise alkoholate oder tertiäre Amine. Besonders bevorzugte Mischungen von Wasserstoffspendermolekül und Base sind Mischungen von Ameisensäure und Triethylamin wie insbesondere das Azeotrope Gemisch daraus, sowie Mischungen von Kaliumisopropylat und Isopropanol.

Die Menge der eingesetzten Metallverbindung oder des eingesetzten Übergangsmetallkomplexes kann bezogen auf den jeweiligen Metallgehalt beispielsweise 0.001 bis 20 mol-%, bezogen auf das eingesetzte Substrat betragen, bevorzugt sind 0.001 bis 2 mol-%, ganz besonders bevorzugt 0.001 bis 1 mol-%.

In einer bevorzugten Ausführungsform können asymmetrische Hydrogenierungen beispielsweise so durchgeführt werden, dass der Katalysator in situ aus einer Metallverbindung und einer Verbindung der Formel (I) gegebenenfalls in einem geeigneten organischen Lösungsmittel erzeugt wird, das Substrat zugegeben wird und die Reaktionsmischung bei Reaktionstemperatur entweder unter Wasserstoffdruck gesetzt oder mit einer Mischung aus einem anderen Wasserstoffspendermolekül und einer Base versetzt wird.

Die erfindungsgemäßen Katalysatoren eignen sich insbesondere in einem Verfahren zur Herstellung von Wirkstoffen von Arzneimitteln und Agrarchemikalien, oder Zwischenprodukten dieser beiden Klassen.

Der Vorteil der vorliegenden Erfindung liegt darin, dass unter Verwendung von einfach erhältlichen und gefahrlos handhabbaren Verbindungen eine ganze Klasse leistungsfähiger Katalysatoren hergestellt werden können.

## Beispiele

### Beispiel 1:

Synthese von (S,S)-1,2-Bis-(5*H*-dibenzo[a,d]cyclohepten-5-yl)-1,2-diphenylethandiamin

(S,S-DPENtrop$_2$)

Zu 313 mg (S,S)-Diphenylethandiamin (1.47 mmol) in 20 ml $CH_2Cl_2$ wurden bei Raumtemperatur 667 mg 5*H*-Dibenzo[a,d]cylohepten-5-yl-chlorid (2.95 mmol, 2 eq.) gefügt. Nach 10 min bildete sich ein weisser Niederschlag. Die Suspension wurde noch 2 h weitergerührt, eingeengt und der Rückstand wurde mit 10 gew.%iger, wässriger Kaliumcarbonatlösung und $CH_2Cl_2$ extrahiert. Die organische Phase wurde abgetrennt und die wässrige Phase wurde noch zwei Mal mit $CH_2Cl_2$ extrahiert. Die gesammelten organischen Phasen wurden über Natriumsulfat getrocknet und eingeengt. Chromatographie über Silicagel mit Hexan/Diethylether (3:2 Volumen) und anschließend Diethylether als Eluentien ergab 846 mg DPENtrop$_2$ (1.43 mmol, 97%) als farblosen Schaum.

In $CDCl_3$ liegt die Verbindung in einer symmetrischen (ca. 68%) und einer asymmetrischen (ca. 32%) Konformation vor.

Symmetrische Form:$^1$H-NMR (300 MHz, $CDCl_3$): $\delta$ = 2.48 (d, *J*= 7.8 Hz, 2H, N*H*), 3.09 (s, 2H, *C*HN$_{Trop}$), 4.49 (d, *J* = 7.8 Hz, 2H, C*H*(Ph)(NH$_{Trop}$)), 6.36 (d, *J* = 11.8 Hz, 2H, C*H*$_{olefin}$), 6.72 (d, *J* = 11.8 Hz, 2H, C*H*$_{olefin}$), 6.83-7.47 (m, 26H, C*H*$_{aryl}$). $^{13}$C-NMR (75 MHz, $CDCl_3$): $\delta$ = 65.4 (2C, *C*HN$_{Trop}$), 66.4 (2C, *C*H(Ph)(NHTrop)), 126.7 (2C, *C*H$_{aryl}$), 126.8 (2C, *C*H$_{aryl}$), 126.9 (2C, *C*H$_{aryl}$), 127.6 (2C, *C*H$_{aryl}$), 128.0 (4C, *C*H$_{aryl}$), 128.2 (4C, *C*H$_{aryl}$), 128.6 (2C, *C*H$_{aryl}$), 129.3 (2C, *C*H$_{aryl}$), 129.8 (2C, *C*H$_{aryl}$), 129.9 (2C, *C*H$_{aryl}$), 130.0 (2C, *C*H$_{aryl}$), 130.2 (2C, *C*H$_{olefin}$), 130.3 (2C, *C*H$_{olefin}$), 133.6 (2C, C$_{quart}$), 133.8 (2C, C$_{quart}$), 139.3 (2C, C$_{quart}$), 140.1 (2C, C$_{quart}$), 141.0 (2C, C$_{quart}$). IR: $\nu$ = 3317 m, (NH), 3020 m, 1599 w, 1489 m, 1451 s, 1436 s, 1071 m, 797 s, 760 s, 697 s. MS (70 eV, m/z, %): 538 (44), 476 (50), 296 (27), 207 (18), 191 (100).

Ausgewählte Daten der asymmetrischen Form: $^1$H-NMR (300 MHz, $CDCl_3$): $\delta$ = 2.78 (br, 1H, NH), 3.13 (br, 1H, NH), 3.37 (d, *J*= 7.0 Hz, 1H, C*H*(Ph)(NHTrop)), 3.74 (s, 1H, C*H*N$_{Trop}$), 3.74 (d, *J* = 7.0 Hz, 1H, C*H*(Ph)(NHTrop)), 4.74 (d, *J* = 5.1 Hz, 1H, C*H*N$_{Trop}$), 6.82-7.38 (m, 25H, C*H*$_{aryl}$) 7.7 (d, *J* = 7.8 Hz, 1 H, C*H*$_{aryl}$).

### Beispiel 2:

Synthese von (R,R)-1,2-Bis-(5*H*-dibenzo[a,d]cyclohepten-5-yl)-1,2-diaminocydohexan

(R,R-DACHtrop$_2$)

Zu 264 mg des Weinsäuresalzes von (R,R)-Diaminocyclohexan (1.00 mmol) in 15 ml $CH_2Cl_2$ wurden in einem Scheidetrichter 20 ml gesättigte wässrige Kaliumcarbonat-Lösung gegeben. Die Mischung wurde kräftig geschüttelt und die organische Phase wurde abgetrennt. Die wässrige Phase wurde noch zwei Mal mit 5 ml $CH_2Cl_2$ extrahiert, die gesammelten organischen Phasen wurden über Natriumsulfat getrocknet und filtriert. Zu dieser Lösung wurden 3 ml Triethylamin und dann 454 mg 5*H*-Dibenzo[a,d]cylohepten-5-yl-chlorid (2.00 mmol) gefügt. Die Lösung wurde 15

min bei Raumtemperatur gerührt und dann für 5 min zum Sieden erhitzt. Dabei fiel ein weisser Niederschlag aus. Abziehen des Lösungsmittels im Vakuum ergab einen farblosen Feststoff, der in 50 ml $CH_2Cl_2$ und 20 ml 10 Gew. %iger, wässriger Kaliumcarbonatlösung aufgenommen wurde. Die organische Phase wurde abgetrennt und die wässrige Phase wurde noch zwei Mal mit 20 ml $CH_2Cl_2$ extrahiert. Die gesammelten organischen Phasen wurden über Natriumsulfat getrocknet, filtriert und im Vakuum eingeengt. Chromatographie über Silicagel mit Hexan/Diethylether (1:1 Volumen) und anschließend Diethylether als Eluentien ergab 444 mg DACHtrop$_2$ (0.90 mmol, 90%) als farblosen Feststoff.

Symmetrische Form: $^1$H-NMR (300 MHz, $CDCl_3$): δ = 0.54-1.21 (m, 4H, C$H_{2cyc}$), 1.37-1.72 (m, 4H, C$H_{2cyc}$), 2.0 (m, 2H, C$H_{cyc}$), 2.3 (br, 2H, N*H*), 4.8 (s, br, 2H, C*H*N$_{Trop}$), 6.6 (d, *J* = 11.7 Hz, 2H, C$H_{olefin}$), 6.8 (d, *J* = 11.7 Hz, 2H, C$H_{olefin}$), 7.4 (m, 16H, C$H_{aryl}$).

Asymmetrische Form: $^1$H-NMR (300 MHz, $CDCl_3$): δ = 0.63-2.34 (m, 10H, C$H_{2cyc}$ und C$H_{cyc}$), 1.95 (br, 1H, N*H*), 2.85 (br, 1H, N*H*), 4.21 (d, *J*= 3.7 Hz, 1H, C*H*N$_{Trop}$), 5.12 (br, 1H, C*H*N$_{Trop}$), 7.11-7.66 (m, 20H, C$H_{Trop}$ und C$H_{olefin}$), $^{13}$ C-NMR (75 MHz, $CDCl_3$): δ = 24.9-32.1 (8C, *C*$H_{2cyc}$), 56.2 (*C*HN$_{Trop, asym}$), 59.5 (*C*HN$_{cyc, asym}$), 59.8 (*C*HN$_{cyc, asym}$), 60.3 (2C, *C*HN$_{cyc, sym}$), 67.1 (*C*HN$_{Trop, asym}$), 67.7 (2C, *C*HN$_{Trop, sym}$), 122.7-134.3 (40C, *C*H$_{aryl}$ und *C*H$_{olefin}$), 131.5-139.0 (16C, *C*$_{quart}$).

IR: ν = 3302 m, (NH), 3014 m, 2919 m, 1486 m, 1436 s, 1101 m, 798 s, 764 s, 729 s. MS (70 eV, m/z, %): 494 (1), 303 (61), 191 (100), 96 (20).

**Beispiel 3:**

Synthese von [Ir(Cl)(CO)(S,S-DPENtrop$_2$)]

Eine Suspension von 29.6 mg S,S-DPENtrop$_2$ (0.05 mmol) aus Beispiel 1 und 15.5 mg [Ir(CO)$_3$(Cl)] (0.05 mmol) in $CD_3CN$ (0.45 ml) wurden für 3 h auf 60°C erwärmt. Beim Abkühlen kristallisierten 31 mg [Ir(Cl)(CO)(DPENtrop$_2$)] (0.036 mmol, 72 %) aus.

Alternative Synthese: Zu 119 mg DPENtrop$_2$ (0.20 mmol) und 67 mg [Ir(COD)(Cl)]$_2$ (0.10 mmol) unter einer Atmosphäre von Kohlenmonoxid wurden 5 ml Acetonitril gefügt. Dabei bildete sich eine gelbgrüne Lösung, die nach 1 h Rühren bei RT filtriert und im Vakuum eingeengt wurde. Der Rückstand wurde aus Toluol umkristallisiert und ergab 127 mg [Ir(Cl)(CO)(DPENTrop$_2$)] (0.15 mmol, 75%).

$^1$H-NMR (300.1 MHz, $CD_2Cl_2$): δ = 3.18 (dd, $^3J_{HH}$ = 11.3 Hz, $^3J_{HH}$ = 11.0 Hz, 1H, C*H*(NH)(Ph)), 3.35 (d, $^3J_{HH}$ = 8.3 Hz, 1H, C$H_{olefin}$), 3.80 (d, $^3J_{HH}$ = 8.3 Hz, 1H, C$H_{olefin}$), 4.29 (dd, $^3J_{HH}$ = 11.4 Hz, $^3J_{HH}$ = 11.3 Hz, 1H, C*H*(NH)(Ph)), 4.39 (d, $^3J_{HH}$ = 2.0 Hz, 1H, C*H*N$_{trop}$), 5.50 (dd, *J* = 11.2 Hz, *J* = 2.0 Hz, 1H, N*H*), 5.87 (dd, *J* = 11.0 Hz, *J* = 10.6 Hz, 1H, N*H*), 5.90 (s, br, 2H, C$H_{ar}$), 6.44 (d, *J* = 7.1 Hz, 1H, C$H_{ar}$), 6.46 (d, *J* = 11.7 Hz, 1H, C$H_{olefin}$), 6.67 (d, *J* = 10.6 Hz, 1H, C*H*N$_{trop}$), 6.65-6.71 (m, 2H, C$H_{ar}$), 6.79-6.85 (m, 3H, C$H_{ar}$), 7.05 (ddd, *J* = 7.6 Hz, *J* = 7.6 Hz, *J* = 1.2 Hz, C$H_{ar}$), 7.11-7.51 (m, 15H, C$H_{ar}$), 7.16 (d, *J* = 11.7 Hz, 1H, C$H_{olefin}$), 7.61 (d, *J* = 7.6 Hz, 1H, C$H_{ar}$), 7.95 (d, *J* = 7.6 Hz, 1H, C$H_{ar}$). - $^{13}$C-NMR (75.5 MHz, $CD_2Cl_2$): δ = 26.4 (*C*H$_{trop}$), 28.0 (*C*H$_{trop}$), 64.1 (*C*HN$_{trop}$), 70.4 (*C*HN$_{trop}$), 71.2 (CH(Ph)N), 73.5 (CH(Ph)N), 124.9 (*C*H$_{ar}$), 125.2 (*C*H$_{ar}$), 126.3 (*C*H$_{ar}$), 127.2 (*C*H$_{ar}$), 127.7 (2C, *C*H$_{ar}$), 127.9 (*C*H$_{ar}$), 128.2 (*C*H$_{ar}$), 128.3 (*C*H$_{ar}$), 128.5 (*C*H$_{ar}$), 128.6 (*C*H$_{ar}$), 128.9 (3C, *C*H$_{ar}$), 128.9 (*C*H$_{olefin}$), 129.0 (*C*H$_{ar}$), 129.0 (*C*H$_{ar}$), 129.1 (*C*H$_{ar}$), 129.1 (*C*H$_{ar}$), 129.2 (*C*H$_{ar}$), 129.4 (*C*H$_{ar}$), 129.7 (*C*H$_{ar}$), 130.9 (*C*H$_{olefin}$), 131.5 (*C*H$_{ar}$), 133.2 (2 *C*$_{quart}$), 133.9 (*C*$_{quart}$), 135.2 (*C*$_{quart}$), 136.2 (*C*$_{quart}$), 136.5 (*C*$_{quart}$), 137.9 (*C*$_{quart}$), 138.9 (*C*$_{quart}$), 139.1 (*C*$_{quart}$), 141.2 (*C*O); (Rotation eines Phenylsubstituenten um die *C*$_{ipso}$-CH(Ph)N Bindung führt zu stark verbreiterten Resonanzen für die *ortho*- und *meta*-Kohlenstoffe). IR: ν = 3271 m, 3171 w, 3020 w, 1975 vs (CO), 1489 m, 1454 m, 1422 w, 1260 w, 1084 w, 987 m, 936 m, 797 m, 732 s.

**Beispiel 4:**

Katalytische Transferhydrierung mit [Ir(Cl)(CO)(S,S-DPENtrop$_2$)]

Zu einer Lösung von 0.01 mmol [Ir(Cl)(CO)(DPENtrop$_2$)] aus Beispiel 3 in 10 ml 2-Propanol wurden 120 mg Acetophenon (1 mmol) und 11 mg Kalium-tert-butanolat (0.1 mmol) gefügt, und die Mischung wurde für 1 h auf 80°C erwärmt. Gaschromatographie der Reaktionslösung (chirale Säule Machery-Nagel Lipodex-E) zeigte vollständigen Umsatz von Acetophenon zu (R)-1-Phenylethanol. Das Produkt wird mit einem Enantiomerenüberschuss von 82% ee erhalten.

**Patentansprüche**

1. Verbindungen der Formel (I),

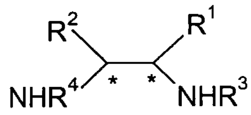

(I)

in der

* ein stereogenes Kohlenstoffatom markiert, das (S)- oder (R)-konfiguriert sein kann

$R^1$ und $R^2$ jeweils unabhängig voneinander ausgewählt sind aus der Gruppe $C_1$-$C_{12}$-Alkyl, $C_1$-$C_{12}$-Alkoxy, $C_1$-$C_{12}$-Halogenalkoxy, $C_1$-$C_{12}$-Halogenalkyl, $C_4$ -$C_{14}$-Aryl, $C_5$-$C_{15}$-Arylalkyl oder zusammen stehen für $C_3$-$C_{12}$-Alkylen oder $C_3$-$C_{12}$-Alkenylen,

$R^3$ und $R^4$ jeweils unabhängig voneinander für Reste der Formel (II) stehen

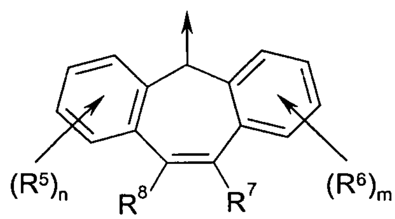

(II)

wobei

die Pfeile jeweils die Bindung des gesamten Restes zum Stickstoffatom anzeigen oder, sofern sie in die Mitte eines aromatischen Systems zeigen, eine Bindung der jeweiligen Reste zum aromatischen Gerüst in beliebiger Position,

$R^5$ und $R^6$ jeweils unabhängig voneinander ausgewählt sind aus der Gruppe Fluor, Chlor, Brom, Iod, Nitro, freies oder geschütztes Formyl, $C_1$-$C_{12}$-Alkyl, $C_1$-$C_{12}$-Alkoxy, $C_1$-$C_{12}$-Halogenalkoxy, $C_1$-$C_{12}$-Halogenalkyl, $C_4$-$C_{14}$-Aryl, $C_5$-$C_{15}$-Arylalkyl oder Resten der Formel (IV),

L-Q-T-W (IV)

in der unabhängig voneinander

L fehlt oder für $C_1$-$C_8$-Alkylen oder $C_2$-$C_8$-Alkenylen steht und

Q fehlt oder für Sauerstoff, Schwefel oder $NR^9$ steht,

T für eine Carbonyl-Gruppe steht und

W für $R^9$, $OR^9$, $NHR^9$ oder $N(R^{10})_2$ steht,
wobei $N(R^{10})_2$ weiterhin als Ganzes für einen 5 oder 6 gliedrigen cyclischen Aminorest stehen kann oder Resten der Formeln (Va-g)

| | | | |
|---|---|---|---|
| L-W | (Va) | L-$SO_2$-W | (Vb) |
| L-$NR^{12}SO_2R^{12}$ | (Vc) | L-$SO_3Z$ | (Vd) |
| L-$PO_3Z_2$ | (Ve) | L-COZ | (Vf) |
| L-CN | (Vg) | | |

in denen L, Q, W und $R^{10}$ die unter der Formel (IV) angegebene Bedeutung besitzen und Z für

Wasserstoff oder M steht und

$R^7$ und $R^8$ jeweils unabhängig voneinander stehen für Wasserstoff, Cyano, Fluor, Chlor, Brom, Iod, $C_1$-$C_{18}$-Alkyl, $C_4$-$C_{24}$-Aryl, $C_5$-$C_{25}$-Arylalkyl, $CO_2M$, wobei M für ein Alkalimetallion oder ein gegebenenfalls organisches Ammoniumion stehen kann, $CONH_2$, $SO_2N(R^9)_2$, wobei $R^9$ für Wasserstoff, $C_1$-$C_{12}$-Alkyl, $C_4$-$C_{14}$-Aryl oder $C_5$-$C_{15}$-Arylalkyl steht, $SO_3M$ oder für Reste der Formel (III),

$$\text{T-Het-R}^{10} \qquad \text{(III)}$$

in der

T fehlt oder für Carbonyl steht,

H et für Sauerstoff oder $NR^9$ steht,

$R^{10}$ für $C_1$-$C_{18}$-Alkyl, $C_4$-$C_{24}$-Aryl oder $C_5$-$C_{25}$-Arylalkyl oder $N(R^{10})_2$ als Ganzes für einen 5 oder 6 gliedrigen cyclischen Aminorest steht und

n und m jeweils unabhängig voneinander für 0, 1, 2 oder 3 stehen.

2. Verfahren zur Herstellung von Verbindungen nach Anspruch 1, **dadurch gekennzeichnet, dass** Verbindungen der Formel (VI)

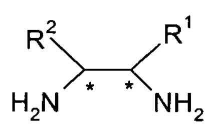

(VI)

in der $R^1$ und $R^2$ die im Anspruch 1 genannte Bedeutung besitzen
mit Verbindungen der Formel (VII) umgesetzt werden,

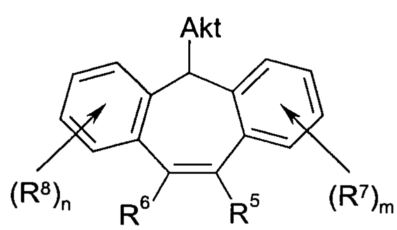

(VII)

in der $R^5$, $R^6$, $R^7$, $R^8$, n und m im Anspruch 1 genannten Bedeutungen besitzen und
Akt für Chlor, Brom, Iod, Trifluoracetyl oder einen Sulfonyloxy-Rest steht,
die dabei entstehenden Ammoniumsalze der Formeln (VIIIa) und/oder (VIIIb) und/oder (VIIIc)

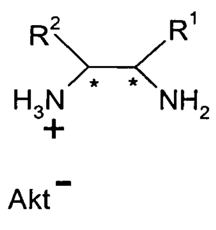

(VIIIa)

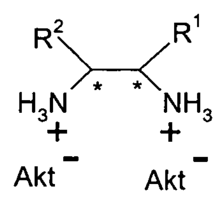

(VIIIb)

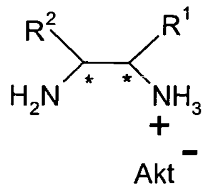 (VIIIb)

in Gegenwart von Base entweder in situ oder in einem nachgeschalteten Reaktionsschritt in Verbindungen der Formel (I) gemäß Anspruch 1 überführt werden.

**3.** Verbindungen der Formeln (VIIIa), (VIIIb) und (VIIIc) gemäß Anspruch 2.

**4.** Übergangsmetallkomplexe von Verbindungen der Formel (I) gemäß Anspruch 1.

**5.** Übergangsmetallkomplexe gemäß Anspruch 4, **dadurch gekennzeichnet, dass** es Übergangsmetallkomplexe von Ruthenium, Osmium, Cobalt, Rhodium, Iridium, Nickel, Palladium, Platin und Kupfer sind.

**6.** Übergangsmetallkomplexe gemäß Anspruch 4 oder 5, **dadurch gekennzeichnet, dass** sie aus Verbindungen der Formel (I) gemäß Anspruch 1 und einer Metallverbindung erzeugt wurden, oder Übergangsmetallkomplexe, die aus den Verbindungen der Formel (I) und einer Metallverbindung im Reaktionsmedium erzeugt werden.

**7.** Übergangsmetallkomplexe gemäß Anspruch 4, 5 oder 6, **dadurch gekennzeichnet, dass** sie der Formel (Xa), (Xb) oder(Xc) gehorchen

$$[M^5(I)]An \quad (Xa)$$

$$[M^5(I)(CO)Hal]An \quad (Xb)$$

$$[Ir(I)(Diolefin)]An \quad (Xc)$$

in denen jeweils

$M^5$ für Rhodium oder Iridium

(I) für eine Verbindung der Formel (I)

Diolefin für ein Dien wie beispielsweise 1,5-Cyclooctydien oder Norbornadien und

An ohne Rücksicht auf mögliche Koordination an das Metallatom für ein Anion und

Hal für Chlorid, Bromid oder Iodid steht.

**8.** Katalysatoren enthaltend Übergangsmetallkomplexe gemäß Ansprüchen 4, 5, 6 oder 7.

**9.** Verwendung von Katalysatoren gemäß Anspruch 8 für Hydrogenierungen.

Europäisches Patentamt

# EUROPÄISCHER RECHERCHENBERICHT

Nummer der Anmeldung
EP 05 01 1538

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | WO 03/048175 A (EIDGENÖSSISCHE TECHNISCHE HOCHSCHULE ZÜRICH) 12. Juni 2003 (2003-06-12) * das ganze Dokument * ----- | 1,4-9 | C07C211/42 C07C211/63 C07F15/00 B01J31/22 C07B53/00 |
| A | DEBLON S ET AL: "HIGHLY DISTORTED D8-RHODIUM(+I) AND D10-RHODIUM(-I) COMPLEXES: SYNTHESIS, REACTIVITY, AND STRUCTURES IN SOLUTION AND SOLID STATE" NEW JOURNAL OF CHEMISTRY, CNRS-GAUTHIER-VILLARS, MONTROUGE, FR, Bd. 25, Nr. 1, 2001, Seiten 83-92, XP008015055 ISSN: 1144-0546 * das ganze Dokument * ----- | 1,4-9 | |
| A | THOMAIER J ET AL: "DIBENZOTROPYLIDENE PHOSPHANES (TROPPS): SYNTHESIS AND COINAGE METAL COMPLEXES" NEW JOURNAL OF CHEMISTRY, CNRS-GAUTHIER-VILLARS, MONTROUGE, FR, Bd. 22, Nr. 9, 1998, Seiten 947-958, XP008015151 ISSN: 1144-0546 * das ganze Dokument * ----- | 1,4-9 | RECHERCHIERTE SACHGEBIETE (Int.Cl.7) C07C C07F B01J C07B |
| A | TURECEK, F.: "Stereochemical interactions in ammonium dications, hypervalent diammonium cation-radicals and ammonium radicals. A B3-MP2 computational study" EUROPEAN JOURNAL OF MASS SPECTROMETRY , 9(4), 267-277 CODEN: EJMSCL; ISSN: 1469-0667, 2003, XP008050841 * das ganze Dokument * ----- -/-- | 3 | |

Der vorliegende Recherchenbericht wurde für alle Patentansprüche erstellt

| Recherchenort | Abschlußdatum der Recherche | Prüfer |
|---|---|---|
| Den Haag | 10. August 2005 | Beslier, L |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

1

EPO FORM 1503 03.82 (P04C03)

Europäisches Patentamt

# EUROPÄISCHER RECHERCHENBERICHT

Nummer der Anmeldung
EP 05 01 1538

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | AERTS, J.: "An improved molecular modeling method for the prediction of enantioselectivity" JOURNAL OF COMPUTATIONAL CHEMISTRY , 16(7), 914-22 CODEN: JCCHDD; ISSN: 0192-8651, 1995, XP008050845 * das ganze Dokument * ----- | 3 | |
| A | REETZ, M. T. ET AL: "Enantiotopic Group Recognition: Direct Evidence for Selective Complexation of Enantiotopic Groups by a Chiral Host" JOURNAL OF THE AMERICAN CHEMICAL SOCIETY , 118(18), 4494-5 CODEN: JACSAT; ISSN: 0002-7863, 1996, XP002339935 * das ganze Dokument * ----- | 3 | |
| | | | RECHERCHIERTE SACHGEBIETE (Int.Cl.7) |

Der vorliegende Recherchenbericht wurde für alle Patentansprüche erstellt

| Recherchenort | Abschlußdatum der Recherche | Prüfer |
|---|---|---|
| Den Haag | 10. August 2005 | Beslier, L |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

1
EPO FORM 1503 03.82 (P04C03)

## ANHANG ZUM EUROPÄISCHEN RECHERCHENBERICHT ÜBER DIE EUROPÄISCHE PATENTANMELDUNG NR. EP 05 01 1538

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-08-2005

| Im Recherchenbericht angeführtes Patentdokument | Datum der Veröffentlichung | Mitglied(er) der Patentfamilie | Datum der Veröffentlichung |
|---|---|---|---|
| WO 03048175 A | 12-06-2003 | DE 10159015 A1 | 12-06-2003 |
| | | AU 2002358570 A1 | 17-06-2003 |
| | | CN 1599743 A | 23-03-2005 |
| | | WO 03048175 A1 | 12-06-2003 |
| | | EP 1448575 A1 | 25-08-2004 |
| | | JP 2005511702 T | 28-04-2005 |
| | | US 2005107608 A1 | 19-05-2005 |

EPO FORM P0461

Für nähere Einzelheiten zu diesem Anhang : siehe Amtsblatt des Europäischen Patentamts, Nr.12/82